# EUROPÄISCHE PATENTSCHRIFT

(11) **EP 3 934 711 B1**
(45) Veröffentlichungstag und Bekanntmachung des Hinweises auf die Patenterteilung: **26.11.2025**
(21) Anmeldenummer: 20710450.6
(22) Anmeldetag: 04.03.2020
(51) Int. Cl.: A61M 1/34, A61M 1/36

(54) **EXTRAKORPORALES BLUTLEITUNGSSET UND BLUTBEHANDLUNGSMASCHINE**
EXTRACORPORAL BLOOD LINE SET AND BLOOD TREATMENT MACHINE
ENSEMBLE DE CONDUITS SANGUINS ET MACHINE DE TRAITEMENT SANGUIN EXTRACORPORELS

(30) Priorität: 06.03.2019 DE 102019105655
(43) Veröffentlichungstag der Anmeldung: 12.01.2022
(73) Patentinhaber: B. Braun Avitum AG, 34212 Melsungen (DE)
(72) Erfinder: JANIK, Waldemar, 34212 Melsungen (DE)
(74) Vertreter: Winter, Brandl - Partnerschaft mbB
(86) Internationale Anmeldenummer: PCT/EP2020/055605
(87) Internationale Veröffentlichungsnummer: WO 2020/178301

(56) Entgegenhaltungen:
- FR-A1- 2 639 831
- US-A- 3 807 401
- US-A- 4 540 406
- US-A- 4 551 131

## Beschreibung

Die vorliegende Offenbarung betrifft eine extrakorporale Blutbehandlungsmaschine, insbesondere eine Dialysemaschine.

### Hintergrund der Erfindung

Bei der extrakorporalen Blutbehandlung (z.B. Hämodialyse) wird das Blut des Patienten von der sogenannten Dialysierflüssigkeit im Dialysator umspült. Das Blut wird hierbei durch ein sogenanntes arterielles Schlauchsystem vom Patienten zum Dialysator gefördert und anschließend über ein venöses Schlauchsystem zurück zum Patienten geleitet. Die Blutpumpe befindet sich i.d.R. im arteriellen Schlauchsystem.

Dem Patientenblut wird vor dem Eintritt in den Dialysator (teil-)kontinuierlich Heparin oder ein alternatives Antikoagulationsmedikament (z.B. Citrat) beigemischt, um eine Zusetzung (Clotting) des Dialysators zu verhindern. Dies geschieht beispielsweise mit einer Spritzenpumpe, die das entsprechende Medikament enthält. Die Spritzenpumpe ist dabei häufig eine feste Komponente der Dialysemaschine.

### Stand der Technik

Herkömmliche Dialysemaschinen weisen für die Beimengung des Antikoagulationsmittels häufig eine Spritzenpumpe auf, welche als externe Spritzenpumpe ausgeführt oder als integraler Teil in der Dialysemaschine integriert sein kann. Üblicherweise ist die Spritzenpumpe über einen separaten Schlauch mit einem Luer-Lock-Anschluss mit dem Blutkreislauf verbunden. In der DE 10 2011 008 856 A1 ist beispielsweise ein Schlauchsystem einer Dialysemaschine offenbart, welches eine Konnektionsstelle zur Zuleitung von Heparin und eine gesonderte Konnektionsstelle zur Zuleitung von Citrat aufweist. Beide Antikoagulationsmittel werden dabei jeweils über eine eigene Pumpen- oder Spritzenvorrichtung dem Blutschlauchsystem zugeführt.

Ähnlich dazu, ist in der DE 42 30 513 C1 eine Vorrichtung zur Entfernung von Aluminiumionen aus Blut offenbart. Auch hier kann dem Blutkreislauf zur Vermeidung der Zusetzung des Dialysators das Antikoagulationsmittel über eine zusätzliche Pumpe zugeführt werden.

Die DE 10 2017 210 134 A1 wiederum offenbart ein System zur extrakorporalen Blutbehandlung. Neben der Blutpumpe zur Förderung des Blutstroms ist hier eine zweite zusätzliche Pumpe angeordnet, um dem Blutkreislauf das Antikoagulationsmittel, insbesondere die Citrat-Lösung, zuzuführen.

Die FR 2 639 831 A1 offenbart eine Vorrichtung zur Infusion von Medikamenten, insbesondere Heparin, in den Unterdruckbereich eines extrakorporalen Blutkreislaufs, insbesondere eines extrakorporalen Blutkreislaufs einer Dialysevorrichtung, mit einem Vorratsbehälter für die Medikamente und einer Zuführleitung vom Vorratsbehälter zum extrakorporalen Blutkreislauf.

### Kurzbeschreibung der Erfindung

Ausgehend von dem bekannten Stand der Technik zeigt sich jedoch der Nachteil, der Spritzenpumpe als relativ komplexe und teure Komponente. Des Weiteren beansprucht die Spritzenpumpe sehr viel Platz sowohl innerhalb der Dialysemaschine als auch außerhalb der Dialysemaschine an der Maschinenfront.

Es ist also die Aufgabe der Erfindung, die Nachteile aus dem Stand der Technik zu vermeiden oder wenigstens zu mildern. Insbesondere soll ein Blutleitungsset bereitgestellt werden, welches ein Antikoagulationsmittel kostengünstig und zuverlässig während einer Dialysetherapie unter Wegfall einer herkömmlichen Spritzenpumpe verabreichen kann. Dabei ist es von besonderer Bedeutung für die Patientensicherheit, dass die Verabreichung des Antikoagulationsmittels auch bei Wegfall der Spritzenpumpe mit exakt festgelegter Abgabemenge (Massenstrom) erfolgt.

Diese Aufgabe wird bei einer extrakorporalen Blutbehandlungsmachine gemäß Anspruch 1 gelöst. Weitere optionale Merkmale werden in den abhängigen Ansprüchen 2-5 beansprucht.

Die arterielle Blutleitung des Blutleitungssets einen Abschnitt mit einer Strömungsquerschnittsverengung, einen Abschnitt mit konstantem, verengten Strömungsquerschnitt und einen Abschnitt mit einer Strömungsquerschnittserweiterung auf. Die Fluidzuführleitung ist dabei erfindungsgemäß an dem Abschnitt mit konstantem Strömungsquerschnitt angeschlossen. Wenn nun Blut durch die Blutleitung strömt, wird dieses über den Abschnitt mit der Strömungsquerschnittsverengung beschleunigt, wobei der Druck in der Blutleitung abnimmt. Entlang des Abschnitts mit konstantem Strömungsquerschnitt strömt das Blut mit im Wesentlichen konstantem Unterdruck, so dass Fluid über die Fluidzuführleitung angesaugt werden kann. Aufgrund des konstanten Unterdrucks in dem Abschnitt mit konstantem Strömungsquerschnitt kann die Saugwirkung und damit die Abgabemenge des Antikoagulationsmittels exakt bestimmt und eingestellt werden.

Dies hat den Vorteil, dass das medizinische Fluid dem Blutstrom in der arteriellen Blutleitung durch Ausnutzung des Venturi-Effekts zugeführt werden kann und keine zusätzliche Pumpe zur Förderung der medizinischen Lösung notwendig ist.

Gemäß Erfindung ist eine Fluidzuführleitungs-Ventileinrichtung zwischen dem zumindest einen Anschlussabschnitt und dem Behälteranschluss oder dem Fluidbehälter angeordnet. Diese ist dafür ausgebildet, den Fluidzufuhrstrom zu regeln oder zu steuern, welcher ausschließlich unter Ausnutzung eines durch den Blutstrom in der arteriellen Blutleitung erzeugten Saugdrucks (Unterdrucks) in der Fluidzuführleitung bewirkt wird. Die erfindungsgemäße Blutbehandlungsmaschine erlaubt somit eine regelbare Zufuhr eines medizinischen Fluids in den Blutstrom, ohne eine zusätzliche Pumpe zu benötigen.

Gemäß Erfindung ist der Anschlussabschnitt stromauf der Pumpe zwischen dem Patientenzugang und der Pumpe an der arteriellen Blutleitung angeordnet. Wenn der Anschlussabschnitt an dieser Stelle angeordnet ist, kann der Anschlussabschnitt auch als T-Stück mit im Wesentlichen konstanten Querschnitt in der Blutstromrichtung ausgeführt sein, was die Fertigungskosten bei der Herstellung des Anschlussabschnitts mindert.

In einer bevorzugten Ausgestaltung kann die Steuervorrichtung die Fluidzuführleitungs-Ventileinrichtung basierend auf dem Blutstrom, dem Druck des Blutstroms und einer Solldurchflussmenge eines in dem Fluidbehälter lagernden medizinischen Fluids durch Öffnen der Fluidzuführleitungs-Ventileinrichtung für eine vorbestimmte Öffnungsdauer und bei einer vorbestimmten Öffnungsfrequenz steuern. Somit kann die Durchflussmenge bzw. die Zugabemenge des medizinischen Fluids genau gesteuert werden. Dabei kann die Steuervorrichtung die Öffnungsdauer und die Öffnungsfrequenz durch Verwendung einer Kodierung, insbesondere einem Barker-Code, bestimmen. In einer bevorzugten Ausgestaltung kann ein Barker-Code der Länge drei mit der Kodierung "+1 +1 -1" verwendet werden, wobei "+1" für eine vergleichsweise lange Öffnungsdauer des Ventils mit kurzer Öffnungsfrequenz bzw. langer Periodendauer stehen kann und "-1" dagegen für eine kürzere Öffnungsdauer mit höherer Öffnungsfrequenz bzw. kürzerer Periodendauer stehen kann.

Erfindungsgemäß kann die Blutbehandlungsmaschine eine Detektionseinheit aufweisen, welche einen Verbindungszustand der Fluidzuführleitung mit der Fluidzuführleitungs-Ventileinrichtung erfasst und abhängig von dem Erfassungsergebnis ein, beispielsweise optisches, akustisches oder kapazitives, Signal ausgibt. Dadurch kann ein Nichteinlegen der Fluidzuführleitung und eine damit einhergehende dauerhafte Öffnung der Fluidzuführleitungs-Ventileinrichtung erfasst werden und eine ungewollt hohe und möglicherweise patientengefährdende Verabreichung des medizinischen Fluids verhindert werden.

In einer weiteren erfindungsgemäßen Ausgestaltung kann an der Fluidzuführleitung ein, vorzugsweise nichtinvasiver, Durchflussmengensensor, insbesondere ein Ultraschallsensor, zur Bestimmung einer Lösungsdurchflussmenge in der Fluidzuführleitung angeordnet sein. Dabei kann der Ultraschallsensor integral mit der Fluidzuführleitungs-Ventileinrichtung ausgebildet sein und gleichermaßen die Lösungsdurchflussmenge und den Verbindungszustand der Fluidzuführleitung mit der Fluidzuführleitungs-Ventileinrichtung erfassen. Die Anbringung des Durchflussmengensensors erlaubt eine genaue Überwachung der Durchflussmenge des medizinischen Fluids und die Integration der Durchflussmengenüberwachung und der Verbindungszustandsdetektion ermöglicht eine Verringerung der Komponentenanzahl und der Systemkomplexität. Zusätzlich hat die Positionierung des Durchflussmengensensors auch den Vorteil, dass Luft in der Fluidzuführleitung detektiert werden kann und somit ein mögliches Leerlaufen eines das medizinische Fluid lagernden Behälters festgestellt werden kann.

Erfindungsgemäß kann an der Fluidzuführleitung zusätzlich eine Filtereinheit, insbesondere ein sogenannter Airstop-Filter, angeordnet sein, welche ein Einströmen von Luft in die Fluidzuführleitung verhindert. Dabei kann die Filtereinheit entweder als fester Bestandteil in der Fluidzuführleitung integriert sein oder als ein separates Zwischenstück ausgebildet sein.

Alternativ zur Position des Durchflussmengensensors an der Fluidzuführleitung kann sich der Durchflussmengensensor in einer weiteren erfindungsgemäßen Ausgestaltung stromab der Konnektierungsstelle an der arteriellen Blutleitung befinden. Dabei kann die Durchflussmengenüberwachung durch Verwendung einer Differenz einer ersten Blutdurchflussmenge und einer zweiten Blutdurchflussmenge unter Berücksichtigung der Öffnungsdauer der Fluidzuführleitungs-Ventileinrichtung erfolgen. Bei geschlossener Fluidzuführleitungs-Ventileinrichtung kann der Durchflussmengensensor die erste Blutdurchflussmenge messen und bei geöffneter Fluidzuführleitungs-Ventileinrichtung kann der Durchflussmengensensor die zweite Blutdurchflussmenge messen. Dies hat den Vorteil, dass der Durchflussmengensensor neben der Bestimmung der Durchflussmenge des Fluids auch für eine Detektion möglicherweise in dem Blutstrom enthaltener Luft verwendet werden kann.

In einer weiteren erfindungsgemäßen Ausgestaltung kann die Konnektierungsstelle über eine lösbare Verbindung, insbesondere eine Luer-Lock-Verbindung, an der arteriellen Blutleitung anbringbar sein. Dabei ist die Konnektierungsstelle einschließlich der Fluidzuführleitung als separate Komponente ausgeführt, was den Vorteil hat, dass konventionelle Leitungssets nicht geändert werden müssen.

Alternativ zu der Fluidzuführleitungs-Ventileinrichtung, welche lediglich in dem geschlossenen und offenen Zustand betrieben werden kann, kann bei einer erfindungsgemäßen Ausgestaltung auch ein Proportionalventil verwendet werden, welches durch beliebig genaue Änderung der Querschnittsfläche der Fluidzuführleitung die Durchflussmenge des medizinischen Fluids steuern kann.

### Kurzbeschreibung der Figuren

Fig. 1 ist eine Darstellung zur Veranschaulichung eines Systemaufbaus einer Dialysemaschine gemäß einem ersten Ausführungsbeispiel der vorliegenden Offenbarung;
Fig. 2 ist eine schematische Detailansicht eines Anschlussabschnitts der Dialysemaschine gemäß dem ersten Ausführungsbeispiel;
Fig. 3 ist eine Darstellung zur Veranschaulichung eines Systemaufbaus einer Dialysemaschine gemäß einer Modifikation des ersten Ausführungsbeispiels der vorliegenden Offenbarung; Diese Maschine ist nicht Teil der beanspruchten Erfindung.
Fig. 4 ist eine Darstellung zur Veranschaulichung eines Systemaufbaus einer Dialysemaschine gemäß einem Beispiel der vorliegenden Offenbarung. Diese Maschine ist nicht Teil der beanspruchten Erfindung

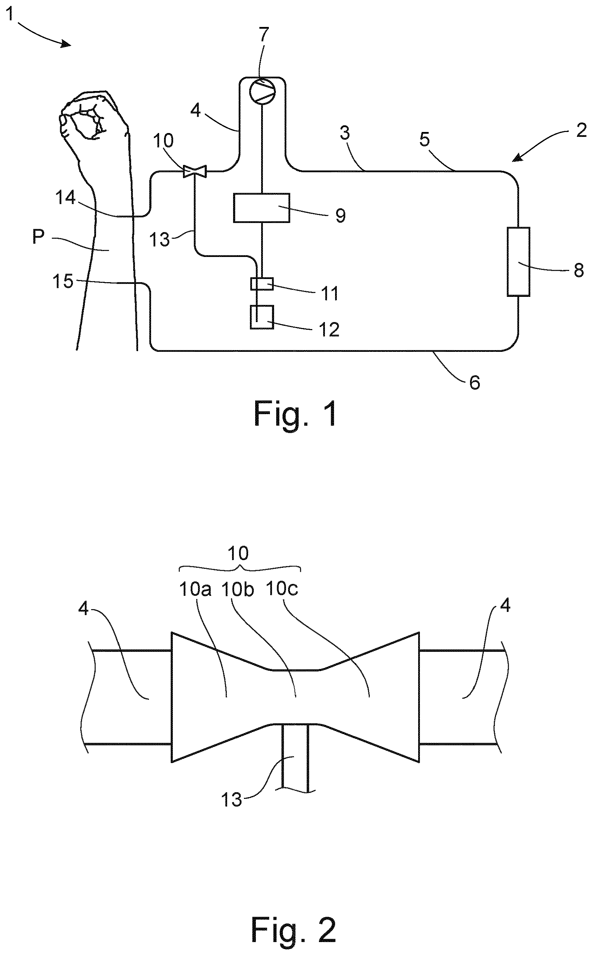

Fig. 1 ist eine Darstellung zur Veranschaulichung eines schematischen Aufbaus einer Dialysemaschine 1 mit einem extrakorporalen Blutleitungssets 2 gemäß einem Ausführungsbeispiel der vorliegenden Offenbarung. Das Leitungsset 2 beinhaltet dabei eine arterielle Blutleitung 3, welche einen ersten arteriellen Blutleitungsabschnitt 4 und einen zweiten arteriellen Blutleitungsabschnitt 5 umfasst, eine venöse Blutleitung 6, eine Pumpe 7, einen Dialysator 8, eine Steuervorrichtung 9, eine/einen Abzweigstelle/Konnektierungsstelle/Anschlussabschnitt 10, ein Ventil 11, ein Behältnis 12 und eine Fluidzuführleitung 13. Der Dialysator 8 ist hier ein Beispiel für "eine Vorrichtung" und dient der Reinigung des Bluts eines Patienten P.

An einem Ende des ersten arteriellen Blutleitungsabschnitts 4 ist ein Leitungsseteinlass 14 ausgebildet, über welchen das zu reinigende Blut des Patienten P in das Leitungsset 2 einströmt und welcher einem ersten Patientenzugang entspricht. Das andere Ende des ersten arteriellen Blutleitungsabschnitts 4 ist mit einer Einlassseite der Pumpe 7 verbunden bzw. in Pumpeneingriff. Eine Auslassseite der Pumpe 7 ist mit einem Ende des zweiten arteriellen Blutleitungsabschnitts 5 verbunden. Im Fall einer für Blutpumpen üblichen Rotor-/Peristaltikpumpe kann die arterielle Blutleitung 3 auch in die Blutpumpe 7 eingeschlauft bzw. eingefädelt sein, wodurch sich der erste und zweite arterielle Blutleitungsabschnitt 4, 5 (einstückig) ergibt. Das andere Ende des zweiten arteriellen Blutleitungsabschnitts 5 ist wiederum mit einem Einlass des Dialysators 8 verbunden. Ein Auslass des Dialysators 8 steht mit einem Ende der venösen Blutleitung 6 in Verbindung. Über einen Leitungssetauslass 15, welcher an dem anderen Ende der venösen Blutleitung 6 ausgebildet ist und einem weiteren Patientenzugang entspricht, wird das gereinigte Blut wieder dem Patienten P zugeführt. Die Anschlüsse, über welche der zweite arterielle Blutleitungsabschnitt 5 und die venöse Blutleitung 6 jeweils mit dem Dialysator 8 verbunden sind, sind in dem bevorzugten Ausführungsbeispiel jeweils als sogenannte Luer-Lock-Verbindungen ausgeführt.

In dem bevorzugten Ausführungsbeispiel ist die Pumpe 7 als Peristaltikpumpe ausgebildet, so dass das Patientenblut aufgrund der Rotation der Pumpe 7 von dem Patienten P zu dem Dialysator 8 gefördert wird. Wenn die Pumpe 7 aufgrund eines Steuerbefehls der Steuervorrichtung 9 betrieben wird, bildet sich in dem ersten arteriellen Blutleitungsabschnitt 4 ein Unterdruck, so dass das zu reinigende Blut des Patienten P angesaugt wird und über den Leitungsseteinlass 14 in den ersten arteriellen Blutleitungsabschnitt 4 einströmt. Die Pumpe 7 fördert das Blut durch den zweiten arteriellen Blutleitungsabschnitt 5 hin zu dem Dialysator 8. Im Dialysator 8 wird das Blut von einer Dialysierflüssigkeit umströmt und dabei gereinigt. Durch den Betrieb der Pumpe 7 bildet sich in der Blutstromrichtung stromab der Pumpe 7 in dem zweiten arteriellen Blutleitungsabschnitt 5, dem Dialysator 8 und der venösen Blutleitung 6 ein Überdruck aus, der das Blut von der Pumpe 7 durch den zweiten arteriellen Blutleitungsabschnitt 5, den Dialysator 8 und die venöse Blutleitung 6 zurück zum Patienten P strömen lässt.

Des Weiteren ist in dem extrakorporalen Leitungsset 2 eine Konnektierungsstelle 10 ausgebildet. Diese Konnektierungsstelle 10 ist zwischen dem Leitungsseteinlass 14 und der Pumpe 7 an dem ersten arteriellen Blutleitungsabschnitt 4 angeordnet und ist im Wesentlichen in Form eines Venturi-Rohrs/einer Venturi-Düse ausgestaltet, so dass sich ein Querschnitt der Konnektierungsstelle 10 entlang der Blutstromrichtung verringert. An einer Stelle, bei welcher der Querschnitt der Konnektierungsstelle 10 minimal ist, ist ein Ende der Fluidzuführleitung 13 abzweigend angeordnet. Die Konnektierungsstelle 10 gemäß dem Ausführungsbeispiel ist schematisch in Fig. 2 dargestellt. Wie hierin zu erkennen, weist die in Form der Venturi-Düse ausgestaltete Konnektierungsstelle 10 in Strömungsrichtung einen Strömungsquerschnittsverengungsabschnitt 10a, einen Abschnitt mit konstantem Strömungsquerschnitt 10b und einen Strömungsquerschnittserweiterungsabschnitt 10c auf, wobei der Strömungsquerschnittsverengungsabschnitt 10a und der Strömungsquerschnittserweiterungsabschnitt 10c jeweils mit dem ersten arteriellen Blutleitungsabschnitt 4 in Fluidverbindung stehen. An dem Abschnitt mit konstantem Strömungsquerschnitt 10b ist die Fluidzuführleitung 13 angeschlossen. Wenn nun Blut durch den ersten arteriellen Blutleitungsabschnitt 4 in die Konnektierungsstelle 10 strömt, wird dieses über den Strömungsquerschnittsverengungsabschnitt 10a beschleunigt. Aufgrund dieser Beschleunigung sinkt der Druck in der Blutströmung. Entlang des Abschnitts mit konstantem Strömungsquerschnitts 10b strömt das Blut demzufolge mit konstantem Unterdruck.

Das andere Ende der Fluidzuführleitung 13 ist mit dem Behältnis 12 verbunden, in welchem ein Antikoagulationsmittel gelagert ist. Das Antikoagulationsmittel ist hier ein Beispiel für "ein medizinisches Fluid". Wenn nun die Pumpe 7, wie vorstehend beschrieben, betrieben wird, strömt das Blut durch das Leitungsset 2 und das Antikoagulationsmittel wird aufgrund der Ausgestaltung der Konnektierungsstelle 10 und dem sich dadurch ausbildenden Venturi-Effekt von dem Behältnis 12 zu der Konnektierungsstelle 10 und schließlich in den ersten arteriellen Blutleitungsabschnitt 4 gesaugt. Neben dem Venturi-Effekt strömt das Antikoagulationsmittel auch aufgrund des vorstehend beschriebenen Unterdrucks in dem ersten arteriellen Blutleitungsabschnitt 4 von dem Behältnis 12 über die Konnektierungsstelle 10 in den ersten arteriellen Blutleitungsabschnitt 4.

Zwischen der Konnektierungsstelle 10 und dem Behältnis 12 ist an der Fluidzuführleitung 13 ein Ventil 11 angeordnet. Das Ventil 11 ist hierbei ein Beispiel für "eine Fluidzuführleitungs-Ventileinrichtung" und eingerichtet, als Antwort auf einen Steuerbefehl der Steuervorrichtung 9 die Strömung des Antikoagulationsmittels von dem Behältnis 12 hin zu der Konnektierungsstelle 10 freizugeben oder zu sperren, indem das Ventil 11 geöffnet oder geschlossen wird. In dem ersten Ausführungsbeispiel ist das Ventil 11 als Quetschventil ausgebildet.

In anderen Worten ausgedrückt, zeigt Fig. 1 exemplarisch den Patienten P, dem durch die arterielle Blutleitung 3 mithilfe der Pumpe 7 Blut entzogen wird, welches durch die Blutleitung 3 zum Dialysator 8 gelangt. Nach der dortigen Reinigung wird das Blut durch die venöse Blutleitung 6 zurück zum Patienten P gefördert. In der arteriellen Blutleitung 3 befindet sich die Konnektierungsstelle 10, an welcher die Fluidzuführleitung 13 angebracht ist. Die Fluidzuführleitung 13 führt zu dem Behältnis 12, welches das Antikoagulationsmittel enthält. Das Element 11 ist als Ventil 11, vorzugsweise als Quetschventil bzw. Klemme, ausgebildet und ermöglicht bzw. verhindert den Transport des Antikoagulationsmittels durch die Fluidzuführleitung 13. Mit anderen Worten kann über das Ventil 11 der Fluss bzw. die Strömung des Antikoagulationsmittels geregelt werden. Die Konnektierungsstelle 10 weist in Blutstromrichtung die Querschnittsänderung auf. An der engsten Stelle ist die Fluidzuführleitung 13 abzweigend platziert. Gemäß dem Prinzip von Venturi wird durch diese Fluidzuführleitung 13 das Antikoagulationsmittel aus dem Behältnis 12 angesaugt, sobald das Blut durch die arterielle Blutleitung 3 strömt. Zusätzlich zum Venturi-Effekt spielt beim Ansaugen des Antikoagulationsmittels auch der Unterdruck in der arteriellen Blutleitung 3 eine Rolle, der durch die Pumpe 7 hervorgerufen wird.

In dem Leitungsset 2 gemäß dem ersten Ausführungsbeispiel ist zusätzlich integral an dem Ventil 11 eine Detektionseinheit in Form eines Ultraschallsensors 16 (nicht in Fig. 1 gezeigt) angeordnet, welcher optisch einen Verbindungszustand der Fluidzuführleitung 13 mit dem Ventil 11 erfasst und basierend auf dem erfassten Verbindungszustand der Fluidzuführleitung 13 mit dem Ventil 11 ein Signal ausgibt. Der Ultraschallsensor 16 ist dabei mit der Steuervorrichtung 9 verbunden. Wenn die Fluidzuführleitung 13 nicht korrekt mit dem Ventil 11 verbunden ist, gibt der Ultraschallsensor 16 das Signal an die Steuervorrichtung 9 aus, welche daraufhin das Ventil 11 schließt und/oder die Pumpe 7 stoppt, so dass eine ungewollt hohe und unter Umständen patientengefährdende Verabreichung des Antikoagulationsmittels verhindert werden kann. Der Ultraschallsensor 16 ist hier nur ein Beispiel für "eine Detektionseinheit".

Mit anderen Worten, um eine sichere und korrekte Verabreichung des Antikoagulationsmittels zu gewährleisten, kann das Quetschventil 11 in seiner Aufnahme für die Fluidzuführleitung 13 eine Detektionseinheit aufweisen, die beispielsweise optisch, akustisch oder kapazitiv feststellen kann, ob die Fluidzuführleitung 13 korrekt eingelegt wurde. Ein Nichteinlegen der Fluidzuführleitung 13 in das Ventil 11 käme sonst einer dauerhaften Öffnung des Ventils 11 gleich, was die ungewollt hohe und unter Umständen patientengefährdende Verabreichung des Antikoagulationsmittels mit sich bringen würde.

Zur Überwachung der korrekten Verabreichung des Antikoagulationsmittels ist der Ultraschallsensor 16 in dem ersten Ausführungsbeispiel eingerichtet, zusätzlich zur Detektion des Verbindungszustands nichtinvasiv z.B. in Form einer Laufzeitdifferenzmessung eine Durchflussmenge des Antikoagulationsmittels in der Fluidzuführleitung 13 zu bestimmen.

Wenn der Ultraschallsensor 16 integral an dem Ventil 11 ausgebildet ist, kann dieser auch Luft in der Fluidzuführleitung 13 detektieren. Wenn durch den Betrieb der Pumpe 7 das Antikoagulationsmittel nach einer gewissen Zeit vollständig aus dem Behältnis 12 geströmt ist, kann Luft in die Fluidzuführleitung 13 gesaugt werden. Um eine Luftinfusion in das Blut des Patienten P zu verhindern, schließt die Steuervorrichtung 9 das Ventil 11, sobald der Ultraschallsensor 16 Luft in der Fluidzuführleitung 13 erfasst. Alternativ oder zusätzlich kann dann auch die Pumpe 7 gestoppt werden.

In anderen Worten ausgedrückt, sieht die Erfindung gemäß dem Ausführungsbeispiel vor, den Durchfluss des Antikoagulationsmittels zu bestimmen. Bevorzugt wird dies durch eine nichtinvasive Durchflussmessung an der Fluidzuführleitung 13 gelöst. Denkbar ist hierbei eine Ultraschalldurchflussmessung in Form einer Laufzeitdifferenzmessung. Der Ultraschallsensor 16 ist in dem Ventil 11 integriert. In diesem Fall übernimmt das Ventil 11 die Aufgaben:
- Aufnahme der Fluidzuführleitung 13
- Quetschen der Fluidzuführleitung 13
- Detektion der Fluidzuführleitung 13
- Durchflussbestimmung in der Fluidzuführleitung 13

In dem Ausführungsbeispiel wird somit sowohl für die Durchflussbestimmung als auch für die Detektion des Verbindungszustands der Ultraschallsensor 16 verwendet. Der Ultraschallsensor 16 an der Fluidzuführleitung 13 hat darüber hinaus den Vorteil, dass damit auch Luft in der Fluidzuführleitung 13 detektiert werden kann. Läuft das Behältnis 12 leer, erkennt der Ultraschallsensor 16 Luft in der Fluidzuführleitung 13, woraufhin das Ventil 11 schließt und somit eine Luftinfusion verhindert wird.

Wie bereits vorstehend beschrieben, wird in dem Ausführungsbeispiel die Durchflussmenge des Antikoagulationsmittels durch periodisches Öffnen und Schließen des Ventils 11 von der Steuervorrichtung 9 gesteuert. Das Ventil 11 beinhaltet in dem Ausführungsbeispiel zusätzlich ein federelastisches Element, welches das Ventil 11 in dem geschlossenen Zustand hält, wenn kein Steuerbefehl von der Steuervorrichtung 9 vorliegt. Wenn die Steuervorrichtung 9 bestimmt, dass das Ventil 11 geöffnet werden soll, wird ein Stellglied, vorzugsweise elektromagnetisch, entgegen der Rückstellkraft des federelastischen Elements bewegt, so dass sich das Ventil 11 öffnet.

Die Steuervorrichtung 9 steuert in dem Ausführungsbeispiel die Durchflussmenge des Antikoagulationsmittels basierend auf der eingestellten Pumpleistung der Pumpe 7 über die Steuerung einer Öffnungsdauer und einer Öffnungsfrequenz des Ventils 11. Dabei ist das Produkt aus der Öffnungsdauer und der Öffnungsfrequenz des Ventils 11 proportional zu der Durchflussmenge des Antikoagulationsmittels durch die Fluidzuführleitung 13.

Mit anderen Worten, erfolgt die Zugabe des Antikoagulationsmittels aus dem Behältnis 12 durch die Fluidzuführleitung 13 erfindungsgemäß periodisch und pulsartig durch kurzzeitige Öffnung des Ventils 11. Die Durchflussmenge/Zugabemenge ist dabei abhängig vom eingestellten Blutstrom der Pumpe 7 sowie von der Öffnungsdauer und der Öffnungsfrequenz des Ventils 11. Das Produkt aus der Öffnungsdauer und der Öffnungsfrequenz wird als Tastverhältnis bezeichnet, welches wiederum proportional zu der Zugabemenge ist. Darüber hinaus spielt auch der im ersten arteriellen Blutleitungsabschnitt 4 vorliegende Druck gegebenenfalls eine Rolle, so dass dieser gemäß der vorliegenden Erfindung ebenfalls berücksichtigt werden kann. Druckmessungen finden an herkömmlichen Dialysemaschinen in den Blutleitungen 4, 5 und 6 bereits statt, so dass der Druck problemlos zur genauen Einstellung der Zugaberate herangezogen werden kann. Des Weiteren ist das Ventil 11 standardmäßig durch eine Federrückstellkraft geschlossen, um ein permanentes Ansaugen des Antikoagulationsmittels zu verhindern. Die Zugabe wird von der Steuervorrichtung 9 unter Berücksichtigung des Blutstroms, des Drucks und der Solldurchflussmenge des Antikoagulationsmittels geregelt.

Um ein Abweichen von einer ordnungsgemäßen Funktion des Ventils 11 festzustellen, erfolgt die Zugabe des Antikoagulationsmittels kodiert. In dem ersten Ausführungsbeispiel wird ein Barker-Code der Länge drei mit "+1 +1 -1" von der Steuervorrichtung 9 vorbestimmt und zur Steuerung des Ventils 11 verwendet. Dabei steht "+1" für eine vergleichsweise lange Öffnungsdauer des Ventils 11 mit kurzer Öffnungsfrequenz bzw. langer Periodendauer. "-1" steht dagegen für eine kürzere Öffnungsdauer mit höherer Öffnungsfrequenz bzw. kürzerer Periodendauer, wobei in beiden Fällen das Tastverhältnis gleich ist und damit die Zugabemenge des Antikoagulationsmittels nicht geändert wird.

Im Betrieb erfasst nun der Ultraschallsensor 16 bei der Durchflussmessung, wie vorstehend genannt, eine Durchflussabfolge und vergleicht diese mit dem vorbestimmten Barker-Code. Bei ordnungsgemäßer Funktion des Ventils 11 entspricht die erfasste Durchflussabfolge dem Barker-Code.

Mit anderen Worten, wenn das Ventil 11 zusätzlich den Ultraschallsensor 16 zur Durchflussmessung aufweist, muss der vorstehend genannte Barker-Code bei funktionierendem Ventil 11 ebenfalls detektierbar sein. Das bedeutet bei "+1 +1 -1": "langer Durchfluss, langer Durchfluss, kurzer Durchfluss".

In dem Ausführungsbeispiel wird die Fluidzuführleitung 13 beim Anschluss an das Leitungsset 2 initial automatisch befüllt, um im Anschluss während der Therapie eine Luftinfusion zu vermeiden. Dies geschieht bestenfalls beim sogenannten Priming durch Öffnung des Ventils 11, welches das Ziel verfolgt, das Blutleitungsset 2 vor dem Anschluss an den Patienten P luftfrei zu machen.

### Modifikation des Ausführungsbeispiels

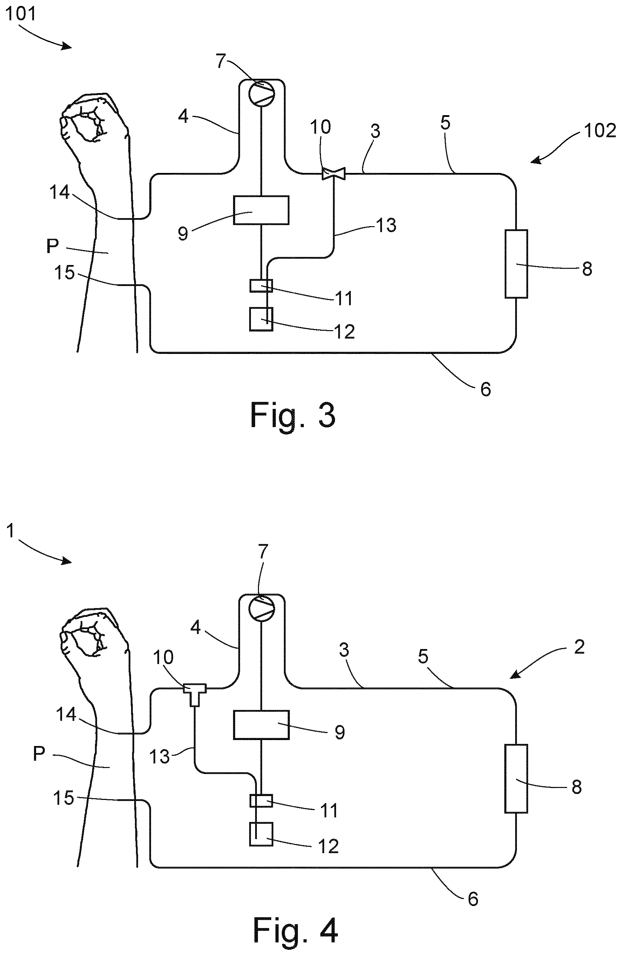

Nachfolgend wird eine Modifikation des Ausführungsbeispiels mit Bezug auf Fig. 3 beschrieben. Fig. 3 zeigt einen Aufbau einer Dialysemaschine 101 mit einem modifizierten extrakorporalen Blutleitungsset 102. Der Aufbau und die Funktionsweise des modifizierten Leitungssets 102 entspricht dabei im Wesentlichen dem extrakorporalen Blutleitungsset 2 gemäß dem Ausführungsbeispiel, weshalb eine Beschreibung davon nicht wiederholt wird und nachfolgend lediglich die Unterschiede beleuchtet werden.

Wie in Fig. 3 zu erkennen, ist in dem Leitungsset 102 gemäß der Modifikation des Ausführungsbeispiels die Konnektierungsstelle 10 an dem zweiten arteriellen Blutleitungsabschnitt 5 zwischen der Pumpe 7 und dem Dialysator 8 angeordnet. Wenn die Konnektierungsstelle 10 in der Blutstromrichtung stromab der Pumpe 7 angeordnet ist, strömt das Antikoagulationsmittel lediglich aufgrund des Venturi-Effekts von dem Behältnis 12 zu der Konnektierungsstelle 10 und in den zweiten arteriellen Blutleitungsabschnitt 5.

In dem modifizierten Ausführungsbeispiel wird folglich die Durchflussmenge des Antikoagulationsmittels im Gegensatz zu dem Ausführungsbeispiel, bei welchem der Druck in dem ersten arteriellen Blutleitungsabschnitt 4 berücksichtigt wird, in Abhängigkeit des im zweiten arteriellen Blutleitungsabschnitt 5 vorliegenden Drucks gesteuert.

Mit anderen Worten, eine nicht beanspruchte, alternative Position für die Venturi-Düse 10 ist im zweiten arteriellen Blutleitungsabschnitt 5 vorgesehen. Da die Düse 10 hierbei stromab der Pumpe 7 platziert ist, herrscht in dem Bereich des zweiten arteriellen Blutleitungsabschnitt 5, in welchem die Düse 10 platziert ist, ein Überdruck, so dass das Ansaugen des Antikoagulationsmittels aus dem Behältnis 12 durch die Fluidzuführleitung 13 allein durch den Venturi-Effekt hervorgerufen wird.

Nachfolgend wird eine in Fig. 4 dargestellte Dialysemaschine 201 beschrieben. Der Aufbau und die Funktionsweise der Dialysemaschine 201 entspricht dabei im Wesentlichen der Dialysemaschine 1 gemäß dem Ausführungsbeispiel, weshalb eine Beschreibung davon nicht wiederholt wird und nachfolgend lediglich die Unterschiede beleuchtet werden.

In diesem nicht beanspruchten Beispiel ist die Konnektierungsstelle/Anschlussabschnitt 210 im Gegensatz zu dem Ausführungsbeispiel als ein einfaches T-Stück mit im Wesentlichen konstantem Querschnitt in Blutstromrichtung ausgebildet. Die Fluidzuführleitung 13 ist dabei abzweigend angeordnet, um das medizinische Fluid dem Blutstrom zuführen zu können. Wenn die Konnektierungsstelle 210 als das T-Stück ausgeführt ist, muss die Konnektierungsstelle 210 in dem ersten arteriellen Blutleitungsabschnitt 4 angeordnet sein. Das Fluid wird dabei lediglich aufgrund des durch die Pumpe 7 in dem ersten arteriellen Blutleitungsabschnitt 4 erzeugten Unterdrucks von dem Behältnis 12 durch die Fluidzuführleitung 13 in den ersten arteriellen Blutleitungsabschnitt 4 gesaugt.

Die vorliegende Erfindung wurde anhand einer beispielhaften Ausführung beschrieben. Allerdings ist die vorliegende Erfindung nicht darauf beschränkt.

In dem Ausführungsbeispiel ist die düsenförmige Konnektierungsstelle 10 fester Bestandteil des Leitungssets 2 in der arteriellen Blutleitung 3. Jedoch kann die Konnektierungsstelle 10 einschließlich der Fluidzuführleitung 13 als zusätzliche separate Komponente (Disposable) ausgeführt sein. So kann die Konnektierungsstelle 10 beispielsweise zwischen dem zweiten arteriellen Blutleitungsabschnitt 5 und dem Dialysator 8 über eine Luer-Lock-Verbindung angebracht werden. Der Vorteil hierbei ist, dass konventionelle Leitungssets nicht geändert werden müssen.

Des Weiteren das Ventil 11 als ein Ventil mit lediglich geöffnetem und geschlossenem Zustand ausgeführt. Als Alternative zu dem Ventil 11 mit nur zwei Zuständen kann jedoch auch ein Proportionalventil verwendet werden. Dieses kann, vorzugsweise durch beliebig genaues Quetschen eines Abschnitts der Fluidzuführleitung 13, den Querschnitt der Fluidzuführleitung 13 ändern und somit die Durchflussmenge des Antikoagulationsmittels steuern. Dabei ist die konkrete Ausführung des Ventils 11 nicht erfindungsrelevant, sondern lediglich die Tatsache, dass mit seiner Hilfe der Fluss des Antikoagulationsmittels durch die Fluidzuführleitung 13 geregelt werden kann.

Der Ultraschallsensor 16 ist als Durchflussmengensensor an der Fluidzuführleitung 13 angeordnet. Jedoch kann auch ein anderer nichtinvasiver Sensor zur Erfassung der Durchflussmenge verwendet werden. Darüber hinaus ist der Ultraschallsensor 16 als Durchflussmengensensor an der Fluidzuführleitung 13 angeordnet. Alternativ kann sich der Durchflussmengensensor auch in der Blutstromrichtung stromab der Konnektierungsstelle 10 des Antikoagulationsmittels an der arteriellen Blutleitung 3 befinden. Ohne Zugabe des Antikoagulationsmittels misst der Sensor dabei eine erste Blutdurchflussmenge. Bei Zugabe des Antikoagulationsmittels misst der Sensor 16 eine zweite Blutdurchflussmenge. Die Differenz aus erster und zweiter Blutdurchflussmenge gibt unter Berücksichtigung der Zugabedauer die Durchflussmenge des Antikoagulationsmittels aus dem Behältnis 12 wieder. Weiter könnte die Zugabemenge des Antikoagulationsmittels auch mittels eines Füllstandssensors im/am oder durch Überwachung des Gewichtes des Behälters 12 ermittelt werden.

Der Ultraschallsensor 16 dient als Detektionseinheit zum Erfassen des Verbindungszustands zwischen der Fluidzuführleitung 13 und dem Ventil 11. Die Detektionseinheit kann jedoch auch durch eine, beispielsweise optische, akustische oder kapazitive, separate Detektionseinheit ausgeführt sein.

Der Ultraschallsensor 16 ist an der Fluidzuführleitung 13 integral an dem Ventil 11 ausgebildet Alternativ kann der Ultraschallsensor 16 auch als eine separate Sensorvorrichtung angeordnet sein.

Der Ultraschallsensor 16 ist zur Detektion eines Leerlaufens des Behältnisses 12 verwendet. Es ist zusätzlich oder als Alternative denkbar, die Fluidzuführleitung 13 mit einem sogenannten Airstop-Filter zu versehen. Diese Filter sind aus der Infusionstechnik hinreichend bekannt und verhindern dort ein Leerlaufen von Infusionssystemen. Ein solcher Filter kann sich zwischen der Fluidzuführleitung 13 und dem Behältnis 12 befinden. Er kann entweder fester Bestandteil der Fluidzuführleitung oder als separates Zwischenstück ausgebildet sein.

Zur Überwachung der ordnungsgemäßen Zugabe des Antikoagulationsmittels wird ein Barker-Code mit der Länge drei verwendet. Jedoch können selbstverständlich auch Barker-Codes anderer Länge oder andere Kodierungen verwendet werden.

Es sei darauf hingewiesen, dass weiter gängige Elemente in Fig. 1 zwar nicht abgebildet sind, dennoch von der Erfindung berücksichtigt werden. Dazu gehören z.B. Luftdetektoren, Luftfallen, Zugabe- und Entnahmestellen, Klemmen bzw. Ventile, nichtoptische Sensoren (z.B. Drucksensoren) und optische Sensoren (z.B. zur Bestimmung des Hämatokrits).

## Patentansprüche

1. Extrakorporale Blutbehandlungsmaschine (1, 101, 201) insbesondere Dialysemaschine mit einem extrakorporalen Blutleitungsset (2, 102, 202), aufweisend
eine arterielle Blutleitung (3) mit einem distalen Patientenzugang und einem proximalen Vorrichtungsanschluss, vorzugsweise Dialysatoranschluss, zwischen denen eine Blutpumpe (7) zum Fördern von Blut aus dem Patienten (P) hin zur Vorrichtung (8), vorzugsweise Dialysator, angeordnet ist,
eine venöse Blutleitung (6) mit einem distalen Patientenzugang und einem proximalen Vorrichtungsanschluss, vorzugsweise Dialysatoranschluss, und
zumindest eine Fluidzuführleitung (13), die an die arterielle Blutleitung (3) in einem Anschlussabschnitt (10, 210) der arteriellen Blutleitung (3), welcher stromauf der Blutpumpe (7) angeordnet angeschlossen ist und an einem Ende einen Behälteranschluss oder einen Fluidbehälter (12) hat,
eine Fluidzuführleitungs-Ventileinrichtung (11) zwischen dem zumindest einen Anschlussabschnitt (10, 210) und dem Behälteranschluss oder dem Fluidbehälter (12), die dafür ausgebildet ist, den Fluidzufuhrstrom zu regeln oder zu steuern, **dadurch gekennzeichnet, dass** der Fluidzufuhrstrom ausschließlich unter Ausnutzung eines durch den Blutstrom in der arteriellen Blutleitung (3, 6) erzeugten Saugdrucks in der Fluidzuführleitung (13) bewirkt wird und dass der Anschlussabschnitt (10,210) in der Form eines Venturi- Rohr ausgebildet ist.

2. Blutbehandlungsmaschine (1, 101, 201) nach Anspruch 1, ferner mit einer Steuervorrichtung (9), welche eingerichtet ist, die Fluidzuführleitungs-Ventileinrichtung (11) basierend auf dem Blutstrom, dem Druck des Blutstroms und einer Solldurchflussmenge eines in dem Fluidbehälter (12) lagernden medizinischen Fluids durch Öffnen der Fluidzuführleitungs-Ventileinrichtung (11) für eine Öffnungsdauer und bei einer Öffnungsfrequenz zu steuern.

3. Blutbehandlungsmaschine (1, 101, 201) nach Anspruch 2, **dadurch gekennzeichnet, dass** die Steuervorrichtung (9) die Öffnungsdauer und die Öffnungsfrequenz durch Verwendung einer Kodierung, insbesondere einem Barker-Code, bestimmt.

4. Blutbehandlungsmaschine (1, 101, 201) nach einem der Ansprüche 1 bis 3, **dadurch gekennzeichnet, dass** die Fluidzuführleitungs-Ventileinrichtung (11) eine Detektionseinheit aufweist, welche beispielsweise optisch, akustisch oder kapazitiv, einen Verbindungszustand der Fluidzuführleitung (13) mit der Fluidzuführleitungs-Ventileinrichtung (11) erfasst und abhängig von dem Erfassungsergebnis ein Signal ausgibt, wenn die Fluidzuführleitung (13) nicht mit der Fluidzuführleitungs-Ventileinrichtung (11) verbunden ist.

## Claims

1. An extracorporeal blood treatment machine (1, 101, 201), in particular a dialyzer, having an extracorporeal blood line set (2, 102, 202), comprising:
an arterial blood line (3) having a distal patient access and a proximal device port, preferably a dialyzer port, between which a blood pump (7) for delivering blood from a patient (P) to the device (8), preferably a dialyzer, is arranged,
a venous blood line (6) having a distal patient access and a proximal device port, preferably a dialyzer port, and
at least one fluid supply line (13) which is connected to the arterial blood line (3) in a port section (10, 210) of the arterial blood line (3) that is connected upstream of the blood pump (7) and at one end includes a container port or a fluid container (12),
a fluid supply line valve unit (11) between the at least one port section (10, 210) and the container port or the fluid container (12) which fluid supply line valve unit is configured to regulate or control the fluid supply flow, **characterized in that** the fluid supply flow is effectuated exclusively using a suction pressure in the fluid supply line (13) generated by the blood stream in the arterial blood line (3, 6) and that the port section (10, 210) is formed in the shape of a Venturi tube.

2. The blood treatment machine (1, 101, 201) according to claim 1, further comprising a control device (9) which is configured to control the fluid supply line valve unit (11) on the basis of the blood stream, the pressure of the blood stream and a target flow rate of a medical fluid stored in the fluid container (12) by opening the fluid supply line valve unit (11) for an opening time and at an opening frequency.

3. The blood treatment machine (1, 101, 201) according to claim 2, **characterized in that** the control device (9) determines the opening time and the opening frequency by using a coding, especially a Barker code.

4. The blood treatment machine (1, 101, 201) according to any of claims 1 to 3, **characterized in that** the fluid supply line valve unit (11) includes a detection unit which detects, for example, optically, acoustically or capacitively, a connection state of the fluid supply line (13) with the fluid supply line valve unit (11), and, in response to the detection result, outputs a signal when the fluid supply line (13) is not connected to the fluid supply line valve unit (11).

## Revendications

1. Machine extracorporelle de traitement du sang (1, 101, 201) en particulier machine de dialyse avec un ensemble de conduites sanguines extracorporelles (2, 102, 202), présentant
une conduite sanguine artérielle (3) avec un accès patient distal et un raccord de dispositif proximal, de préférence un raccord de dialyseur, entre lesquels une pompe à sang (7) est agencée pour le transport du sang du patient (P) vers le dispositif (8), de préférence le dialyseur,
une conduite sanguine veineuse (6) avec un accès patient distal et un raccord de dispositif proximal, de préférence un raccord de dialyseur, et
au moins une conduite d'amenée de fluide (13) qui est raccordée à la conduite sanguine artérielle (3) dans une section de raccord (10, 210) de la conduite sanguine artérielle (3) qui est agencée en amont de la pompe à sang (7) et présente à une extrémité un raccord de récipient ou un récipient de fluide (12),
un dispositif de soupape de conduite d'amenée de fluide (11) entre la au moins une section de raccord (10, 210) et le raccord de récipient ou le récipient de fluide (12) qui est formé afin de réguler ou commander le courant d'amenée de fluide, **caractérisé en ce que** le courant d'amenée de fluide est exclusivement provoqué en utilisant une pression d'aspiration générée par le flux sanguin dans la conduite sanguine artérielle (3, 6) dans la conduite d'amenée de fluide (13) et **en ce que** la section de raccord (10, 210) est formée sous la forme d'un tube Venturi.

2. Machine de traitement du sang (1, 101, 201) selon la revendication 1, de plus avec un dispositif de commande (9) qui est conçu afin de commander le dispositif de soupape de conduite d'amenée de fluide (11) sur la base du flux sanguin, de la pression du flux sanguin et d'une quantité de débit de consigne d'un fluide médical logeant dans le récipient de fluide (12) par ouverture du dispositif de soupape de conduite d'amenée de fluide (11) pendant une durée d'ouverture et à une fréquence d'ouverture.

3. Machine de traitement du sang (1, 101, 201) selon la revendication 2, **caractérisée en ce que** le dispositif de commande (9) détermine la durée d'ouverture et la fréquence d'ouverture par utilisation d'un codage, en particulier d'un code Barker.

4. Machine de traitement du sang (1, 101, 201) selon l'une quelconque des revendications 1 à 3, **caractérisée en ce que** le dispositif de soupape de conduite d'amenée du fluide (11) présente une unité de détection qui détecte par exemple par voie optique, acoustique ou capacitive, un état de liaison de la conduite d'amenée de fluide (13) avec le dispositif de soupape de conduite d'amenée de fluide (11) et émet un signal en fonction du résultat de détection lorsque la conduite d'amenée de fluide (13) n'est pas reliée au dispositif de soupape de conduite d'amenée de fluide (11).
